# FASCICULE DE BREVET EUROPEEN

(11) **EP 1 516 644 B1**
(45) Date de publication et mention de la délivrance du brevet: **28.11.2007**
(21) Numéro de dépôt: 04292248.4
(22) Date de dépôt: 20.09.2004
(51) Int. Cl.: A61M 25/00, A61N 1/05

(54) **Nécessaire de perçage du septum cardiaque et de mise en place d'un dispositif transseptal, notamment d'une sonde de stimulation d'une cavité gauche**
Vorrichtung zum Aufstechen des Septums und Platzieren einer transeptalen Vorrichtung an der linken Seite
Device for piercing the septum and for placing a stimulation transeptal device at the left part

(30) Priorité: 22.09.2003 FR 0311065
(43) Date de publication de la demande: 23.03.2005
(73) Titulaire: ELA MEDICAL, 92541 Montrouge (FR)
(72) Inventeur: Ollivier, Jean-François, 91190 Villiers Le Bacle (FR); D'Hiver, Philippe, 92320 Chatillon (FR)
(74) Mandataire: Dupuis-Latour, Dominique

(56) Documents cités:
- FR-A- 2 365 351
- US-A- 5 190 528
- US-A- 5 312 341
- US-B1- 6 547 787

## Description

L'invention concerne un nécessaire de perçage du septum cardiaque et de mise en place d'un dispositif transseptal.

L'invention peut notamment être utilisée pour la mise en place d'une sonde dans une cavité gauche, en vue de stimuler cette cavité par un "dispositif médical implantable actif" tel que défini par la directive 90/385/CEE du 20 juin 1990 du Conseil des communautés européennes, et plus précisément un dispositif tel qu'un stimulateur cardiaque, défibrillateur et/ou cardioverteur et/ou dispositif "multisite".

Mais l'invention n'est pas limitée à la pose de sondes de stimulation. Comme on le comprendra, elle s'applique avantageusement à toute technique chirurgicale invasive nécessitant la traversée du septum cardiaque, par exemple pour procéder à des investigations cliniques dans l'une ou l'autre des cavités gauches. Il peut s'agir également de procédure d'angioplastie, notamment pour des opérations de valvuloplastie mitrale.

Pour la stimulation des cavités droites, il suffit d'implanter une sonde endocavitaire par le réseau veineux périphérique droit. En revanche, pour stimuler les cavités gauches, la situation est plus complexe et la solution la plus souvent retenue consiste à introduire la sonde par l'oreillette droite puis dans le réseau coronarien via l'ostium du sinus coronaire.

Cette technique d'implantation n'est cependant pas toujours réalisable, notamment lorsque la conformation du sinus coronaire est trop accidentée, ou en case de thrombose.

Une autre solution, dite "approche transseptale", consiste à passer la sonde à travers la cloison interauriculaire ou interventriculaire, ou "septum cardiaque", pour stimuler la cavité gauche - auriculaire et/ou ventriculaire, selon la configuration et le placement de la sonde.

Cette procédure, telle qu'elle est mise en oeuvre actuellement, présente cependant des risques opératoires élevés, notamment de perforation accidentelle de l'aorte, ou encore de dissection des parois de l'oreillette droite par un mouvement rotatoire inopiné de l'aiguille. En tout état de cause, cette technique est très délicate à mettre en oeuvre et requiert une grande habileté du chirurgien qui doit, pour pouvoir traverser le septum, réaliser des piqûres multiples de la paroi, en s'assurant toujours du positionnement parfait de cette dernière, la traversée du septum ne devant être entreprise que s'il ne subsiste aucun doute sur la position de l'aiguille.

Le US-A-5 190 528 décrit un nécessaire permettant d'introduire par voie transseptale un cathéter afin de ponctionner le sang dans la cavité auriculaire gauche, pour le recycler vers le circuit artériel via une pompe extra-corporelle. Le perçage du septum y est réalisé par une aiguille dépassant de l'extrémité d'un cathéter, qui vient percer le septum interatrial pour permettre ensuite le passage du cathéter en faisant avancer ce dernier sur l'aiguille, puis en retirant cette dernière. La manipulation d'un tel dispositif est particulièrement délicate pour éviter toute fausse manoeuvre, et doit être faite par un praticien expérimenté, sous examen fluoroscopique afin de déterminer la position et la distance de l'aiguille par rapport à la paroi à perforer.

Le US-A-5 312 341 décrit un perfectionnement dans lequel le cathéter transseptal est pourvu de moyens de rétention, par exemple sous forme d'un collier gonflable, pour maintenir en place le cathéter de manière que son extrémité distale reste à l'intérieur de l'oreillette gauche. Ce perfectionnement, s'il assure une meilleure sécurité après implantation du cathéter, n'apporte pas de réponse à la difficulté du perçage précis du septum pour la mise en place du cathéter, avec les risques élevés attachés à cette intervention.

L'un des buts de l'invention est de remédier aux difficultés des techniques connues, en proposant un nécessaire de perçage du septum cardiaque et de mise en place d'un dispositif transseptal qui réduise au minimum le caractère invasif de cette intervention (notamment en évitant les piqûres multiples de la paroi), et qui permette un perçage sûr une fois que le site d'intervention sur la paroi du septum a été défini.

On verra par ailleurs que le nécessaire de l'invention est mis en oeuvre par des techniques comparables à des techniques déjà existantes (notamment la pose d'une sonde de stimulation à vis par voie sous-clavière), auquel les praticiens sont rodés, et qu'ils pourront adapter sans difficulté pour une intervention de perçage du septum.

L'invention propose à cet effet un nécessaire comprenant :
- un guide de perçage, comportant un corps de sonde avec : une gaine creuse souple présentant sur toute sa longueur une lumière interne, et comprenant une extrémité proximale apte à recevoir un mandrin d'une série de mandrins successivement introduits par cette extrémité proximale, et une extrémité distale ; et une tête de sonde, disposée à l'extrémité distale de la gaine et traversée de part en part par un alésage axial en communication avec la lumière interne de la gaine, et comportant côté distal une face frontale prolongée par des moyens d'ancrage à la paroi du septum ;
- un mandrin de manipulation, apte à être introduit dans la lumière de la gaine et l'alésage du corps de sonde et mobile en translation par rapport à la sonde jusqu'à une position axiale où ce mandrin ne dépasse pas, côté distal, la face frontale du corps de sonde ;
- un mandrin perceur présentant une extrémité distale pointue, apte à être introduit dans la lumière de la gaine et l'alésage du corps de sonde et mobile en translation par rapport à la sonde jusqu'à une première position axiale où ce mandrin ne dépasse pas, côté distal, la face frontale du corps de sonde puis, par une course axiale additionnelle contrôlée, jusqu'à une seconde position où l'extrémité distale pointue émerge de la face frontale, au travers des moyens d'ancrage, sur une longueur au moins égale à l'épaisseur locale du septum, de manière à traverser celui-ci de part en part ; et
- un mandrin de guidage, apte à être introduit dans la lumière de la gaine et l'alésage du corps de sonde et mobile en translation par rapport à la sonde jusqu'à une position axiale où ce mandrin émerge de la face frontale sur une longueur au moins égale à l'épaisseur locale du septum, de manière à former, après retrait du guide de perçage, un guide axial d'une cavité à l'autre au travers du septum, pour l'introduction dudit dispositif transseptal.

Les moyens d'ancrage peuvent notamment comprendre une vis hélicoïdale solidaire de la tête de sonde, celle-ci étant apte à être entraînée en rotation depuis l'extrémité proximale de la gaine pour permettre l'ancrage de la vis hélicoïdale par vissage dans le tissu du septum.

Le guide de perçage comprend de préférence des premiers moyens de butée, pour limiter le mouvement axial du mandrin de manipulation au-delà d'une position axiale prédéterminée, et/ou des seconds moyens de butée, pour limiter ladite course axiale additionnelle du mandrin perceur.

Ces moyens de butée peuvent notamment être formés d'un rétrécissement du diamètre de l'alésage de la tête de sonde, coopérant avec un renflement du mandrin de manipulation et/ou avec un épaulement du mandrin perceur.

En variante, les moyens de butée peuvent être formés par un taraudage de l'alésage de la tête de sonde, coopérant avec un filetage homologue du mandrin de manipulation et/ou du mandrin perceur - ce qui permet de maîtriser plus finement l'avancée du mandrin perceur.

Dans une forme de réalisation particulière, le guide de perçage comprend un joint perforable, apte à obturer l'alésage du corps de sonde à son débouché dans la face frontale.

Le nécessaire peut également comprendre un cathéter-guide, apte à être introduit dans la gaine creuse souple du guide de perçage, et comportant une lumière interne ouverte à ses deux extrémités, pour recevoir successivement le mandrin perceur puis le mandrin de guidage sur toute la longueur du cathéter-guide et au-delà de l'ouverture distale de celui-ci jusque dans la cavité cardiaque.

Le nécessaire peut alors comprendre avantageusement une poignée de manipulation amovible, comportant un logement interne ouvert latéralement de manière à recevoir simultanément les extrémités proximales de la gaine souple prolongeant le guide de perçage, du cathéter-guide et du mandrin perceur, ce logement interne étant conformé et dimensionné de manière à assujettir entre elles ces extrémités proximales dans des positions relatives prédéterminées, et former ainsi un gabarit de perçage empêchant toute pénétration excessive du mandrin perceur et/ou du cathéter-guide au-delà de la paroi du septum après retrait de la poignée amovible et enfoncement axial du mandrin perceur et du cathéter-guide.

On va maintenant décrire un exemple de mise en oeuvre du dispositif de l'invention, en référence aux dessins annexés.

La figure 1 montre en coupe le guide de perçage de l'invention équipé d'un mandrin de manipulation dans une configuration correspondant à une première phase de l'opération de perçage du septum.

La figure 2 est homologue de la figure 1, le guide de perçage étant équipé d'un mandrin perceur pour une seconde phase de l'opération de perçage.

La figure 3 est homologue de la figure 1, le guide de perçage étant équipé d'un mandrin de guidage pour une troisième phase de l'opération de perçage.

La figure 4 montre le mandrin de guidage de la figure 3 implanté au travers de la paroi du septum, après retrait du guide de perçage, pour un quatrième étape de l'opération de perçage.

Les figures 5 à 7 sont des vues en coupe d'une variante de réalisation du guide de perçage selon l'invention, respectivement dans une configuration d'ancrage du guide, d'approche du mandrin perceur et de traversée de la paroi du septum.

Les figures 8 et 9 illustrent un outil de manipulation utile à l'exécution des différentes étapes de perçage, respectivement dans une configuration initiale et dans une configuration subséquente, une fois le guide de perçage ancré dans la paroi du septum (la figure 9 montrant également sous forme éclatée les différentes pièces de la combinaison d'éléments).

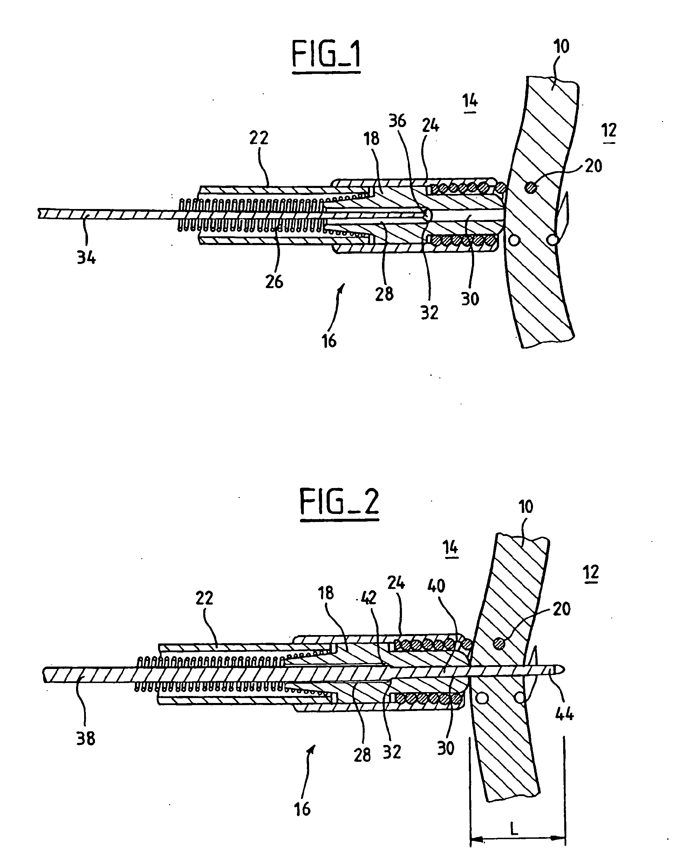

Sur la figure 1, la référence 10 désigne la paroi du septum cardiaque, par exemple, dans l'exemple décrit ci-dessous, la paroi séparant la cavité 12 de l'oreillette gauche de la cavité 14 de l'oreillette droite (on notera cependant que cette description peut être transposée de façon semblable au perçage de la partie du septum cardiaque séparant le ventricule gauche du ventricule droit).

La description qui va suivre sera décrite dans le contexte d'une intervention consistant à passer une sonde à travers la cloison interauriculaire de manière à stimuler une cavité gauche (ventricule ou oreillette) au moyen de cette sonde. Comme on l'a expliqué au début de la présente description, ce type d'intervention n'est cependant pas limitatif, et le nécessaire de l'invention peut être aussi bien utilisé pour d'autres types d'interventions nécessitant le perçage du septum cardiaque, par exemple le passage d'une sonde de mesure de divers paramètres physiologiques depuis l'intérieur d'une cavité gauche, l'introduction d'un cathéter transseptal pour réaliser un accès à une cavité gauche, etc.

Le nécessaire de perçage de l'invention met en oeuvre un guide de perçage 16 dont la structure est comparable à celle d'une sonde de stimulation connue dite "sonde à vis", c'est-à-dire d'une sonde pourvue à son extrémité distale d'une vis hélicoïdale présentant une extrémité affûtée, de manière à permettre l'ancrage dans le tissu cardiaque au point de contact avec ce dernier par un mouvement de rotation imprimé par le praticien depuis l'extrémité proximale et transmis à la tête de sonde, et donc la vis d'ancrage, par la gaine reliant la tête de sonde à l'extrémité proximale.

La méthode d'introduction et de fixation d'une telle sonde est similaire à celle des sondes classiques munies d'une vis d'ancrage.

Plus précisément, la tête de sonde 18 a une forme générale cylindrique, et elle est pourvue côté distal, sur son extrémité frontale de la vis d'ancrage 20 évoquée ci-dessus. Côté opposé, la tête de sonde est reliée à une gaine souple creuse 22 (dont seule une partie a été représentée sur les figures) présentant sur toute sa longueur une lumière interne. La surface extérieure 24 de la tête de sonde est une surface en silicone. La vis d'ancrage 20 est reliée à un conducteur interne 26 logé dans la lumière de la gaine 22 via l'élément 18, permettant ainsi de recueillir un signal électrique au niveau de la paroi du septum.

La tête de sonde 18 présente un alésage axial interne 28 s'étendant jusqu'à la face frontale du corps de sonde et débouchant au centre de celui-ci dans l'axe de la vis d'ancrage hélicoïdale 20. La partie d'extrémité distale 30 de cet alésage présente un diamètre inférieur à celui de la partie 28, de manière à former localement un rétrécissement 32, dont on exposera le rôle ci-dessous.

Dans une première phase de l'intervention, un mandrin de manipulation 34 est introduit dans la lumière de la gaine 22. Ce mandrin de manipulation 34 est du type "mandrin profilé à bille" en lui-même connu, c'est-à-dire que son extrémité distale est effilée et se termine par un renflement 36 évitant tout risque de perçage ou d'accrochage par cette extrémité.

La première phase de l'intervention consiste à mettre en place le mandrin de manipulation 34 dans la lumière de la gaine creuse 22, de manière à rigidifier l'ensemble de la sonde. Le diamètre du renflement 36 étant supérieur à celui de l'alésage axial 30, le renflement 36 vient buter contre le rétrécissement 32, empêchant ainsi que le mandrin de manipulation 34 ne dépasse de la sonde.

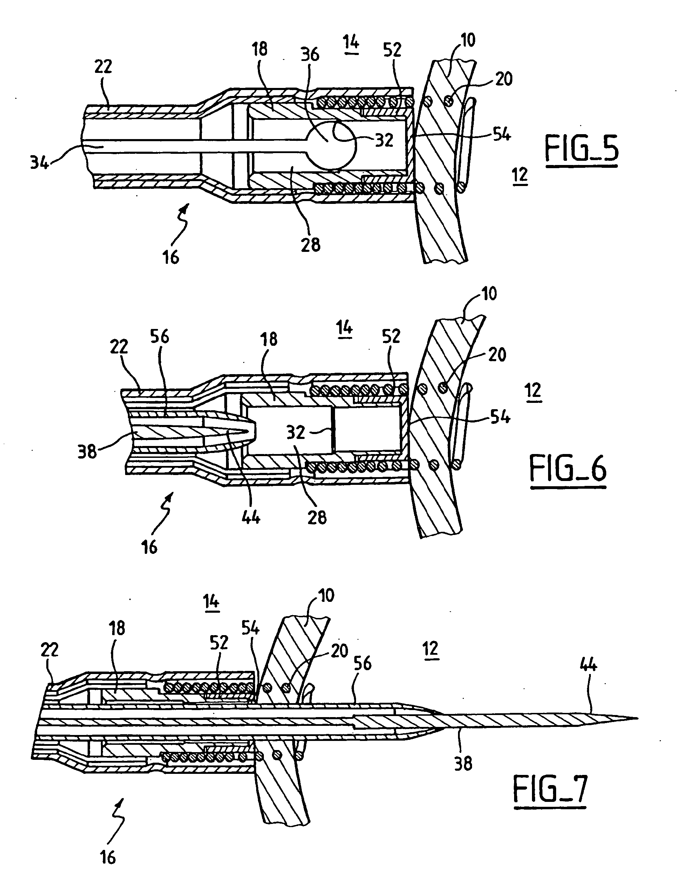

Par des manipulations en elles-mêmes connues, le praticien introduit alors la tête de sonde dans la cavité 14 de l'oreillette droite jusqu'à venir en butée contre la paroi 10 du septum. Il visse ensuite le guide de perçage 16 sur cette paroi 10 en imprimant une rotation au corps de sonde 18, et par voie de conséquence à la vis d'ancrage 20, par l'intermédiaire de la gaine 22 depuis l'extrémité proximale de cette dernière (on décrira plus bas, en référence notamment à la figure 6, un outil permettant de réaliser cette manoeuvre).

Le vissage complet est détecté tactilement par le praticien du fait de la résistance opposée à la rotation ; il est également possible de recueillir un signal électrique caractéristique via la vis 20 afin de s'assurer de la position de la sonde. Cette vérification effectuée, le mandrin de manipulation 34 est retiré et remplacé par un mandrin perceur.

La figure 2 illustre ce mandrin perceur 38, introduit dans le guide de perçage 16.

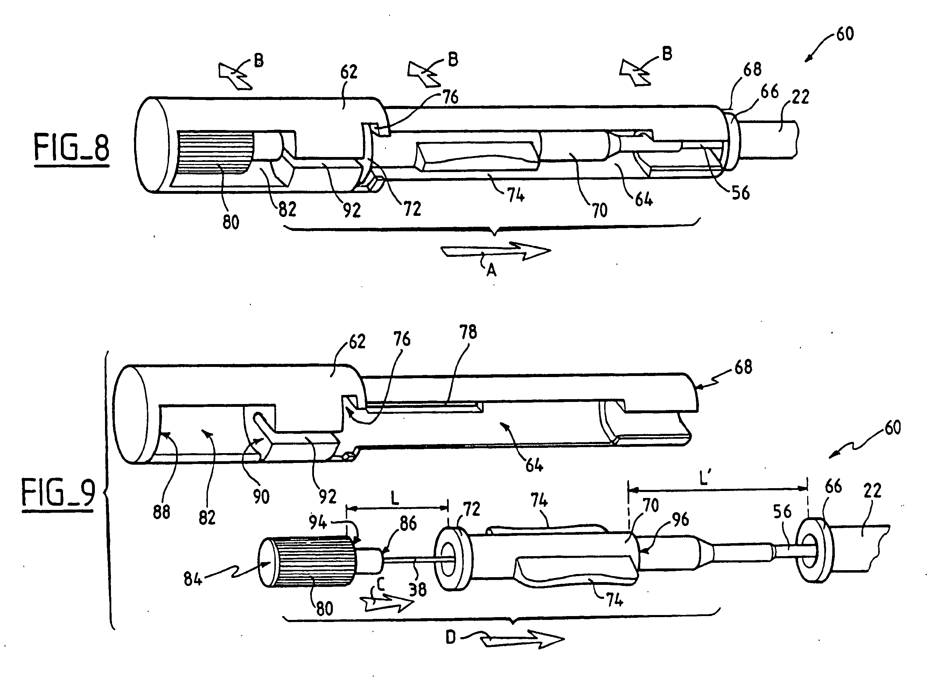

Le mandrin perceur 38 présente une extrémité distale 40 de diamètre réduit, inférieur à celui de l'alésage interne 30 de la tête de sonde. Un épaulement 42 du mandrin perceur vient en butée contre le rétrécissement intérieur 32, à la jonction des parties d'alésage 28 et 30, de manière à limiter la longueur L dont émerge l'extrémité distale pointue 44 du mandrin perceur 38 au-delà de la face frontale de la tête de sonde 18. Lorsqu'il introduit le mandrin perceur 38, le praticien exerce, côté proximal, des poussées répétées sur la poignée de ce mandrin, ce qui a pour effet, à l'autre extrémité, de perforer très localement le septum (on décrira plus bas, en référence notamment à la figure 9, un outil permettant d'effectuer cette manoeuvre).

Du fait du positionnement précis du mandrin perceur 38 par le guide de perçage 16 maintenu en place par la vis d'ancrage 20, l'extrémité pointue 44 reste positionnée précisément dans l'axe de cette vis d'ancrage, ce qui permet de perforer localement et très précisément le septum, sans risque de piqûres multiples ni de dissection de la paroi par un mouvement rotatoire inopiné de l'extrémité pointue, comme cela pouvait être le cas avec les techniques opératoires mises en oeuvre jusqu'à présent.

Une variante (non illustrée) consiste à doter l'extrémité du mandrin perceur 38 d'un filetage coopérant avec un taraudage formé dans l'alésage 30 jusqu'à la butée de l'épaulement 42 : le perçage du septum s'effectue alors avec une rotation relative du mandrin perceur 38 par rapport au guide de perçage 16, ce qui procure l'avantage corrélatif de limiter l'allongement du corps de sonde sous l'effet des contraintes résultant de l'effort de perçage du septum tant que celui-ci n'a pas été traversé par le mandrin perceur 38.

La traversée du septum peut être contrôlée sous amplificateur de brillance. Lorsque la traversée a été effectuée (configuration correspondant à la figure 2), le mandrin perceur 38 est alors retiré et remplacé par un mandrin de guidage 46 ; on notera que le reflux sanguin depuis la cavité gauche est minimal pendant cette opération, car le trou dans le septum est très petit.

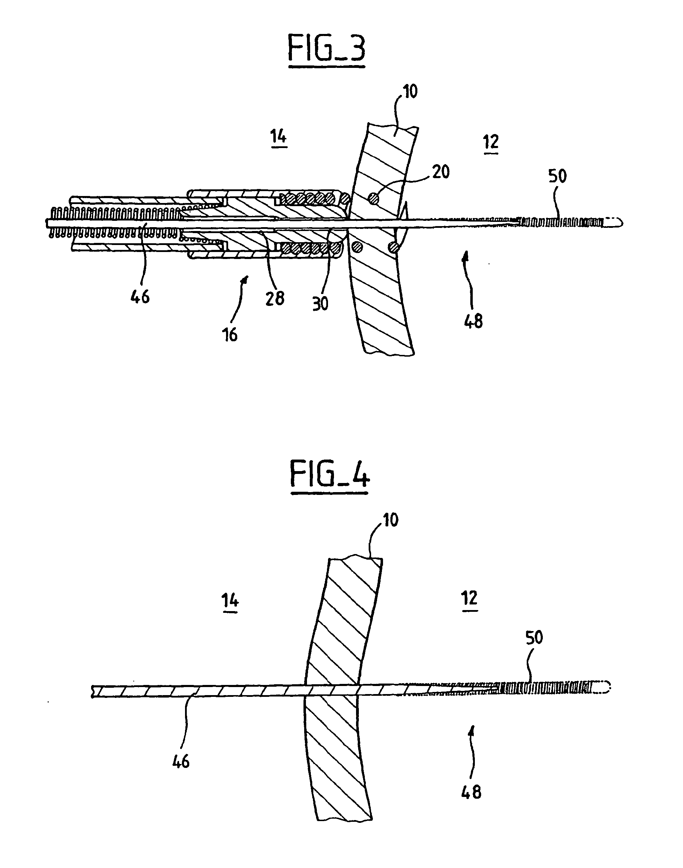

La figure 3 illustre la configuration avec le mandrin de guidage 46 introduit dans le guide de perçage 16 et au travers de la paroi 10.

Le mandrin de guidage 46 est un mandrin d'angioplastie de type classique, c'est-à-dire un mandrin très fin comportant une âme métallique pourvue à son extrémité distale 48 d'un ressort 50 dont l'extrémité souple évite tout risque de perforation. Ce mandrin de guidage 46 est introduit sur une longueur permettant à son extrémité distale 48 de dépasser largement à l'intérieur de la cavité gauche 12.

La phase suivante de l'intervention consiste, tout en maintenant en place le mandrin de guidage 46, à retirer le guide de perçage 16 par un mouvement de rotation inverse, permettant de retirer la vis d'ancrage 20, puis de retrait par une translation arrière guidée par le mandrin 46.

La configuration finale ainsi obtenue est celle illustrée figure 4, avec le mandrin 46 formant un guide axial d'une cavité (oreillette droite 14) à l'autre (oreillette gauche 12) au travers de la paroi du septum 10.

Ce mandrin 46 pourra ensuite être utilisé pour toute opération en elle-même classique, notamment l'introduction d'un cathéter-guide enfilé sur le mandrin 46, éventuellement via un dilatateur, sur toute la longueur de celui-ci (technique dite de "filoguidage" ou "OTW", *Over The Wire).* Le cathéter-guide une fois mis en place au travers du septum et débouchant dans l'oreillette gauche, le mandrin de guidage 46 sera extrait de ce cathéter-guide pour laisser le passage libre à une sonde qui pourra être implantée dans l'oreillette gauche ou le ventricule gauche.

Les figures 5 à 7 illustrent une variante de réalisation du guide de perçage 16, où l'extrémité distale de la tête de sonde 18 est fermée par un couvercle en forme de tube cylindrique 52 dont la face frontale 54 comporte une région centrale perforable mais qui, dans la condition initiale illustrée figure 5, assure une étanchéité entre l'alésage axial interne 28 de la tête de sonde 18 et l'environnement extérieur de manière à éviter tout reflux sanguin vers l'intérieur du guide de perçage 16.

Comme on le comprendra, le mandrin perceur, lorsqu'il aura été substitué au mandrin de manipulation (configuration illustrée figure 7), pourra venir perforer par son extrémité pointue la cloison 54, avec une étanchéité renforcée à cet endroit, tout particulièrement lorsque ce mandrin perceur sera lui-même retiré pour laisser place au mandrin de guidage.

Les figures 5 à 7 sont des vues en coupe d'une variante de réalisation du guide de perçage selon l'invention, respectivement dans une configuration (i) d'ancrage du guide, (ii) d'approche du mandrin perceur et (iii) de traversée de la paroi du septum par le mandrin perceur combiné à un cathéter-guide.

Dans ce mode de réalisation comme dans le précédent, l'alésage axial inteme comporte un léger épaulement 32 (visible également figure 6) servant de butée pour la bille ou le renflement d'extrémité 36 du mandrin de manipulation 34.

En revanche, la lumière de la gaine souple creuse 22 présente, ainsi que l'alésage axial interne 28, un diamètre agrandi par rapport au mode de réalisation précédent, de manière à permettre l'introduction non seulement des mandrins successifs (mandrin de manipulation, mandrin perceur et mandrin de guidage) mais également d'un cathéter-guide 56 (cf. figures 6 et 7), introduit dans le dispositif et au travers de la paroi du septum en même temps que le mandrin perceur.

Plus précisément, la figure 6 illustre la configuration des divers éléments lorsque le cathéter-guide 56 et le mandrin perceur 38, après avoir été introduits dans la gaine soule 22 par l'extrémité proximale de celle-ci, se trouvent à proximité de l'alésage axial interne 28.

L'étape suivante consiste, sans modifier la position du cathéter-guide, à faire avancer le mandrin perceur 38 en direction distale jusqu'à ce que celui-ci vienne perforer par son extrémité pointue 44 la cloison 54, avec une étanchéité renforcée à cet endroit de manière à éviter tout reflux sanguin dans l'alésage interne 28 du guide de perçage 16.

L'étape suivante consiste à faire progresser simultanément en direction distale le mandrin perceur 38 et le cathéter-guide 56, jusqu'à ce que ce dernier traverse la paroi 10 du septum et vienne émerger à l'intérieur de la cavité 12. La configuration des différents éléments à la fin de cette étape est celle illustrée figure 7.

L'étape suivante (en elle-même conventionnelle et non illustrée) consiste, en laissant en place le cathéter-guide 56, à retirer entièrement le mandrin perceur 38, de sorte qu'après ce retrait le cathéter-guide se retrouve seul en place, procurant un passage entre la cavité 12 et l'extérieur du corps du patient. Par ce passage, le chirurgien pourra ensuite introduire le mandrin de guidage jusqu'à ce que ce dernier vienne émerger du cathéter-guide jusque dans la cavité 12. A la fin de cette opération, la configuration sera semblable à celle de la figure 7, mais avec le mandrin de guidage (semblable au mandrin de guidage 46 des figures 3 et 4) substitué au mandrin perceur 38.

Ensuite, le praticien extrait le cathéter-guide. Puis il retire le guide de perçage 16, par un mouvement de rotation inverse, ayant pour effet de dévisser la vis d'ancrage, suivi d'une translation arrière guidée par le mandrin de guidage ; la configuration des différents éléments au cours de ces deux dernières étapes sera semblable à celle illustrée antérieurement sur les figures 3 et 4.

Les figures 8 et 9 illustrent un outil de manipulation permettant d'accomplir de manière précise les différentes étapes de perçage et d'introduction et du cathéter-guide. La figure 8 illustre l'ensemble des différents éléments, référencé 60, dans une configuration initiale où l'outil de manipulation est assujetti au guide de perçage, tandis que la figure 9 illustre ce même ensemble dans une configuration de l'étape subséquente, une fois le guide de perçage ancré dans la paroi du septum - cette figure 9 montrant également sous forme éclatée les différentes pièces de la combinaison.

Cet ensemble 60 comprend une poignée amovible 62, combinée aux différents éléments formant les extrémités proximales de la gaine souple 22 prolongeant le guide de perçage, du cathéter-guide 56 et du mandrin perceur 38. Plus précisément, la poignée amovible 62 comporte un logement interne 64 ouvert latéralement de manière à recevoir les divers éléments précités avec un positionnement axial relatif particulier.

La figure 8 illustre la poignée amovible 62 avec ces différents éléments logés dans la cavité interne 64, tandis que la figure 9 montre ces mêmes éléments dans la position relative qu'ils occupent juste après le retrait de la poignée amovible, celle-ci ayant été simplement éloignée en direction radiale.

La gaine souple 22 du guide de perçage 16 se termine côté proximal par une collerette 66 susceptible de venir en butée contre la face distale 68 de la poignée amovible 62.

Le cathéter-guide 56 émerge de la gaine souple 22 en direction proximale, où il est prolongé par un corps élargi 70 se terminant côté proximal par une collerette 72, ce corps étant pourvu latéralement d'ailettes 74 pour rendre plus facile la manipulation par le praticien. La collerette 72 vient se loger dans un évidement homologue 76 de la poignée amovible 78, et il est prévu dans cette dernière une ouverture 78 pour le passage de l'une des ailettes (l'autre ailette étant tournée du côté de l'ouverture latérale du logement interne 64 de la poignée amovible).

Le mandrin perceur 38, quant à lui, est terminé côté distal par un bouton de manoeuvre 80 pouvant être reçu dans un logement homologue 82 de la poignée amovible 62. Les faces d'extrémités du bouton de manoeuvre 80, viennent se placer, côté proximal 84 et côté distal 86, contre des faces d'extrémité homologues opposées 88 et 90, respectivement, de la cavité 82. Une fente longitudinale 92, ouverte latéralement de la même façon que le logement interne 64, relie les logements 82 et 64 en recevant la partie du mandrin perceur 38 comprise entre le bouton de manoeuvre 80 et la collerette 72 du cathéter-guide 56.

La séquence des opérations est la suivante.

Initialement, l'ensemble se trouve dans la configuration illustrée figure 8, où les différents éléments sont retenus dans une position relative donnée, imposée par la géométrie des cavités de la poignée amovible 62. La position axiale du mandrin perceur 38 et du cathéter-guide 56, entre eux et par rapport au guide de perçage 16, correspond à celle illustrée figure 6, c'est-à-dire que la pointe 44 du mandrin perceur 38 n'émerge pas du cathéter-guide 56, et que ce dernier est en retrait par rapport au plan d'extrémité distale du guide de perçage 16.

Initialement, le mandrin perceur 38 et le cathéter-guide 56 sont assujettis en position l'un par rapport à l'autre au moyen de la poignée amovible 62.

Le chirurgien peut alors introduire cet ensemble dans la gaine souple 22 jusqu'à ce que la face distale 68 de la poignée 62 vienne en butée contre la collerette 66 de la gaine souple 22 (flèche A de la figure 8).

Les différents éléments se présentent alors comme illustré figure 8.

L'étape suivante consiste à retirer la poignée 62, simplement par un déplacement latéral (flèche B de la figure 8).

Les différents éléments se présentent alors comme illustré figure 9.

L'étape suivante consiste à enfoncer le bouton 80 du mandrin perceur (flèche C de la figure 9), permettant ainsi à l'autre extrémité de perforer le septum, et ceci sans modifier la position du cathéter-guide 56. La profondeur de pénétration du mandrin perceur 38 est précisément définie par la cote L définie entre la collerette 72 et la face 94 en vis-à-vis du bouton de manoeuvre 80, cette cote étant définie par la géométrie de la poignée amovible 62 lorsque celle-ci logeait ces deux éléments.

L'étape suivante consiste à enfoncer l'ensemble mandrin perceur-cathéter-guide (flèche D de la figure 9) à l'intérieur de la gaine souple 22 du guide de perçage, de manière à faire progresser simultanément ces deux éléments jusqu'à ce qu'ils viennent émerger tous deux au-delà de la paroi du septum, jusque dans la cavité 12 (c'est-à-dire dans la position illustrée figure 7). Ici encore, la profondeur de pénétration est définie par la cote L' définie entre la collerette 66 de la gaine souple 22 et une face en vis-à-vis 96 du corps 70 prolongeant le cathéter-guide, cette cote étant définie par la géométrie du logement interne de la poignée amovible 62.

Comme on l'aura compris, la poignée amovible 62 permet d'assujettir entre eux les différents éléments (gaine souple 22 solidaire du guide de perçage 16, cathéter-guide 56 et mandrin perceur 38) dans des positions relatives prédéterminées, assurant ainsi une fonction de gabarit de perçage empêchant toute pénétration excessive du mandrin perceur et/ou du cathéter-guide à l'intérieur de la cavité 12 après retrait de la poignée et enfoncement axial du mandrin perceur et du cathéter-guide.

## Revendications

1. Un nécessaire de perçage du septum cardiaque et de mise en place d'un dispositif transseptal, notamment d'une sonde de stimulation d'une cavité gauche, **caractérisé en ce qu'**il comprend :
- un guide de perçage (16), comportant un corps de sonde avec :
· une gaine creuse souple (22) présentant sur toute sa longueur une lumière interne, et comprenant une extrémité proximale apte à recevoir un mandrin d'une série de mandrins successivement introduits par cette extrémité proximale, et une extrémité distale, et
. une tête de sonde (18, 24), disposée à l'extrémité distale de la gaine et traversée de part en part par un alésage axial (28, 30) en communication avec la lumière interne de la gaine, et comportant côté distal une face frontale prolongée par des moyens (20) d'ancrage à la paroi du septum (10) ;
- un mandrin de manipulation (34), apte à être introduit dans la lumière de la gaine et l'alésage du corps de sonde et mobile en translation par rapport à la sonde jusqu'à une position axiale où ce mandrin ne dépasse pas, côté distal, la face frontale du corps de sonde ;
- un mandrin perceur (38) présentant une extrémité distale pointue (44), apte à être introduit dans la lumière de la gaine et l'alésage du corps de sonde et mobile en translation par rapport à la sonde jusqu'à une première position axiale où ce mandrin ne dépasse pas, côté distal, la face frontale du corps de sonde puis, par une course axiale additionnelle contrôlée, jusqu'à une seconde position où l'extrémité distale pointue (44) émerge de la face frontale, au travers des moyens d'ancrage (20), sur une longueur au moins égale à l'épaisseur locale du septum (10), de manière à traverser celui-ci de part en part ; et
- un mandrin de guidage (46), apte à être introduit dans la lumière de la gaine et l'alésage du corps de sonde et mobile en translation par rapport à la sonde jusqu'à une position axiale où ce mandrin émerge de la face frontale sur une longueur au moins égale à l'épaisseur locale du septum, de manière à former, après retrait du guide de perçage (16), un guide axial d'une cavité à l'autre au travers du septum, pour l'introduction dudit dispositif transseptal.

2. Le nécessaire de la revendication 1, dans lequel les moyens d'ancrage comprennent une vis hélicoïdale (20) solidaire de la tête de sonde (18, 24), celle-ci étant apte à être entraînée en rotation depuis l'extrémité proximale de la gaine pour permettre l'ancrage de la vis hélicoïdale par vissage dans le tissu du septum (10).

3. Le nécessaire de la revendication 1, dans lequel le guide de perçage (16) comprend des premiers moyens de butée, pour limiter le mouvement axial du mandrin de manipulation au-delà d'une position axiale prédéterminée.

4. Le nécessaire de la revendication 1, dans lequel le guide de perçage (16) comprend des seconds moyens de butée, pour limiter ladite course axiale additionnelle du mandrin perceur.

5. Le nécessaire de la revendication 3 ou de la revendication 4, dans lequel les premiers et/ou les seconds moyens de butée sont formés d'un rétrécissement (32) du diamètre de l'alésage (28, 30) de la tête de sonde, coopérant avec un renflement (36) du mandrin de manipulation (34) et/ou avec un épaulement (42) du mandrin perceur (38).

6. Le nécessaire de la revendication 3 ou de la revendication 4, dans lequel les premiers et/ou les seconds moyens de butée comprennent un taraudage de l'alésage de la tête de sonde, coopérant avec un filetage homologue du mandrin de manipulation et/ou du mandrin perceur.

7. Le nécessaire de la revendication 1, dans lequel le guide de perçage (16) comprend un joint perforable (52, 56), apte à obturer l'alésage du corps de sonde à son débouché dans la face frontale.

8. Le nécessaire de la revendication 1, comprenant en outre :
- un cathéter-guide (56), apte à être introduit dans la gaine creuse souple (22) du guide de perçage (16), et comportant une lumière interne ouverte à ses deux extrémités, pour recevoir successivement le mandrin perceur (38) puis le mandrin de guidage (46) sur toute la longueur du cathéter-guide et au-delà de l'ouverture distale de celui-ci jusque dans la cavité cardiaque.

9. Le nécessaire de la revendication 7, comprenant en outre :
- une poignée de manipulation amovible (62), comportant un logement interne (64) ouvert latéralement de manière à recevoir simultanément les extrémités proximales de la gaine souple (22) prolongeant le guide de perçage (16), du cathéter-guide (56) et du mandrin perceur (38), ce logement interne (64) étant conformé et dimensionné (L, L') de manière à assujettir entre elles ces extrémités proximales dans des positions relatives prédéterminées, et former ainsi un gabarit de perçage empêchant toute pénétration excessive du mandrin perceur et/ou du cathéter-guide au-delà de la paroi du septum après retrait de la poignée amovible (62) et enfoncement axial du mandrin perceur (38) et du cathéter-guide (56).

## Claims

1. A kit for piercing the cardiac septum and placing a transseptal device, in particular a probe for the stimulation of a left cavity, **characterised in that** it comprises:
- a piercing guide (16) having a probe body including:
- a flexible hollow sheath (22) having an internal lumen over its whole length, and comprising a proximal end able to receive a mandrel out of a series of mandrels successively introduced via this proximal end, and a distal end, and -a probe head (18, 24) arranged at the distal end of the sheath and traversed from end to end by an axial bore (28, 30) in communication with the internal lumen of the sheath, and comprising, at the distal side, a frontal face extended by means (20) for anchoring to the wall of the septum (10);
- a handling mandrel (34) able to be introduced into the lumen of the sheath and the bore of the probe body, and movable in translation relative to the probe up to an axial position where this mandrel does not protrude, at the distal side, from the frontal face of the probe body;
- a piercing mandrel (38) having a pointed distal end (44) able to be introduced into the lumen of the sheath and the bore of the probe body and movable in translation relative to the probe up to a first axial position where this mandrel does not protrude, at the distal side, from the frontal face of the probe body, and then, via a controlled additional axial travel, up to a second position, where the pointed distal end (44) emerges from the frontal face through the anchoring means (20) over a length at least equal to the local thickness of the septum (10) in order to traverse it from one side to the other; and
- a guiding mandrel (46) able to be introduced into the lumen of the sheath and the bore of the probe body and movable in translation relative to the probe up to an axial position where this mandrel emerges from the frontal face over a length at least equal to the local thickness of the septum, so as to form, after withdrawal of the piercing guide (16), an axial guide from one cavity to the other through the septum, for the introduction of said transseptal device.

2. The kit of claim 1, wherein the anchoring means comprise a helical screw (20) connected to the probe head (18, 24), which is able to be rotatably driven from the proximal end of the sheath to allow the anchoring of the helical screw by screwing into the tissue of the septum (10).

3. The kit of claim 1, wherein the piercing guide (16) comprises first abutment means to limit the axial movement of the handling mandrel beyond a predetermined axial position.

4. The kit of claim 1, wherein the piercing guide (16) comprises second abutment means to limit said additional axial travel of the piercing mandrel.

5. The kit of claim 3 or claim 4, wherein the first and/or second abutment means are made of a narrowing (32) of the diameter of the bore (28, 30) of the probe head, which cooperates with a bulge (36) of the handling mandrel (34) and/or with a shoulder (42) of the piercing mandrel (38).

6. The kit of claim 3 or claim 4, wherein the first and/or second abutment means comprise a female thread of the bore of the probe head, which cooperates with a corresponding male thread of the handling mandrel and/or the piercing mandrel.

7. The kit of claim 1, wherein the piercing guide (16) comprises a penetrable seal (52, 56) able to seal off the bore of the probe body at its opening into the frontal face.

8. The kit of claim 1, further comprising:
- a catheter guide (56) able to be introduced into the flexible hollow sheath (22) of the piercing guide (16) and comprising an internal lumen open at its both ends to successively receive the piercing mandrel (38) and then the guiding mandrel (46) over the entire length of the catheter guide and beyond its distal opening up to the interior of the cardiac cavity.

9. The kit of claim 7, further comprising:
- a removable handling grip (62) comprising an internal receptacle (64), which is laterally open so as to receive simultaneously the proximal ends of the flexible sheath (22) extending the piercing guide (16), the catheter guide (56) and the piercing mandrel (38), this internal receptacle (64) being conformed and dimensioned (L, L') so as to fix these proximal ends relative to each other in predetermined relative positions and thus form a piercing device preventing any excessive penetration of the piercing mandrel and/or the catheter guide beyond the wall of the septum after withdrawal of the removable grip (62) and axial pushing in of the piercing mandrel (38) and the catheter guide (56).

## Patentansprüche

1. Ausrüstung zum Durchstechen der Herzscheidewand und zur Platzierung einer transseptalen Vorrichtung, insbesondere einer Sonde zur Stimulation eines linken Herzraums, **dadurch gekennzeichnet, dass** sie folgendes umfasst:
- eine Stichführung (16) mit einem Sondenkörper mit:
einer flexiblen hohlen Hülle (22), die über ihre ganze Länge einen-internen Kanal aufweist und ein nahe gelegenes Ende umfasst, das dazu geeignet ist, einen Dorn aus einer Serie von Dornen aufzunehmen, die nacheinander über dieses Nahe gelegene Ende eingeführt werden, sowie ein entferntes Ende, und
einem Sondenkopf (18, 24), der am entfernten Ende der Hülle angeordnet ist und von einer Seite zur anderen von einer axialen Bohrung (28, 30) durchquert wird, die in Verbindung mit dem internen Kanal der Hülle steht, sowie an der entfernten Seite eine frontale Fläche umfasst, die durch Mittel (20) zur Verankerung an der Wandung der Herzscheidewand (10) verlängert ist;
- einen Manipulierdorn (34), der dazu geeignet ist, in den Kanal der Hülle und die Bohrung des Sondenkörpers eingeführt zu werden, und im Verhältnis zur Sonde bis zu einer axialen Position verschiebbar beweglich ist, wo dieser Dorn am entfernten Ende nicht über die frontale Fläche des Sondenkörpers hervorsteht;
- einen Stechdorn (38) mit einem spitzen entfernten Ende (44), der dazu geeignet ist, in den Kanal der Hülle und die Bohrung des Sondenkörpers eingeführt zu werden, und im Verhältnis zur Sonde bis zu einer ersten axialen Position, wo dieser Dorn am entfernten Ende nicht über die frontale Fläche des Sondenkörpers hervorsteht, verschiebbar beweglich ist, und dann durch einen zusätzlichen gesteuerten axialen Lauf bis zu einer zweiten Position, wo das spitze entfernte Ende (44) über die frontale Fläche austritt, durch die Verankerungsmittel (20) hindurch, über eine Länge, die wenigstens gleich der lokalen Dicke der Scheidewand (10) ist, um diese so von der einen Seite zur anderen zu durchqueren; und
- einen Führungsdorn (46), der dazu geeignet ist, in den Kanal der Hülle und die Bohrung des Sondenkörpers eingeführt zu werden, und im Verhältnis zur Sonde bis zu einer axialen Position verschiebbar beweglich ist, wo dieser Dorn über die frontale Fläche über eine Länge austritt, die wenigstens gleich der lokalen Dicke der Scheidewand ist, um so nach dem Zurückziehen der Stichführung (16) eine axiale Führung von einem Raum zum anderen durch die Scheidewand zu bilden, zur Einführung der transseptalen Vorrichtung.

2. Ausrüstung nach Anspruch 1, bei welcher die Verankerungsmittel eine mit dem Sondenkopf (18, 24) verbundene spiralförmige Schraube (20) umfassen, wobei diese dazu geeignet ist, ausgehend von dem nahe gelegenen Ende der Hülle drehend mitgenommen zu werden, um die Verankerung der spiralförmigen Schraube durch Einschrauben in das Gewebe der Scheidewand (10) zu erlauben.

3. Ausrüstung nach Anspruch 1, bei welcher die Stichführung (16) erste Anschlagmittel umfasst, um die axiale Bewegung des Manipulierdorns über eine vorbestimmte axiale Position hinaus zu begrenzen.

4. Ausrüstung nach Anspruch 1, bei welcher die Stichführung (16) zweite Anschlagmittel umfasst, um den zusätzlichen axialen Lauf des Stechdorns zu begrenzen.

5. Ausrüstung nach Anspruch 3 oder nach Anspruch 4, bei welcher die ersten und/oder die zweiten Anschlagmittel aus einer Verengung (32) des Durchmessers der Bohrung (28, 30) des Sondenkopfes gebildet sind, die mit einer Verdickung (36) des Manipulierdorns (34) und/oder mit einer Schulter (42) des Stechdorns (38) zusammenwirkt.

6. Ausrüstung nach Anspruch 3 oder nach Anspruch 4, bei welcher die ersten und/oder die zweiten Anschlagmittel ein Innengewinde der Bohrung des Sondenkopfs umfassen, das mit einem entsprechenden Außengewinde des Manipulierdorns und/oder des Stechdorns zusammenwirkt.

7. Ausrüstung nach Anspruch 1, bei welcher die Stichführung (16) eine perforierbare Dichtung (52, 56) umfasst, die dazu geeignet ist, die Bohrung des Sondenkörpers an deren Mündung in die frontale Fläche zu verschließen.

8. Ausrüstung nach Anspruch 1, weiterhin mit:
- einer Katheterführung (56), die dazu geeignet ist, in die flexible hohle Hülle (22) der Stichführung (16) eingeführt zu werden, und einen an seinen beiden Enden offenen Innenkanal umfasst, um nacheinander den Stechdorn (38) und dann den Führungsdorn (46) über die gesamte Länge der Katheterführung und jenseits deren entfernten Öffnung bis in den Herzraum aufzunehmen.

9. Ausrüstung nach Anspruch 7, weiterhin mit:
- einem abnehmbaren Manipuliergriff (62), der einen inneren Sitz (64) umfasst, welcher seitlich offen ist, um gleichzeitig das nahe gelegene Ende der die Stichführung (16) verlängernden flexiblen Hülle (22), der Katheterführung (56) und des Stechdorns (38) aufzunehmen, wobei dieser innere Sitz (64) so angepasst und dimensioniert ist (L, L'), dass diese nahe gelegenen Enden untereinander auf vorbestimmte relative Positionen festgelegt sind, um so ein Stechwerkzeug auszubilden, das jegliches übermäßiges Eindringen des Stechdorns und/oder der Katheterführung jenseits der Wandung der Scheidewand nach Entfernen des abnehmbaren Griffs (62) und axialem Eindrücken des Stechdorns (38) und der Katheterführung (56) verhindert.
